# EUROPEAN PATENT APPLICATION

(11) **EP 1 488 804 A1**
(43) Date of publication of application: **22.12.2004**
(21) Application number: 03712773.5
(22) Date of filing: 19.03.2003
(51) Int. Cl.: A61K 39/395

(54) **IMMUNOGLOBULIN/HYDROPHILIC PEPTIDE COMPLEXES**

(30) Priority: 22.03.2002 JP 2002081968
(71) Applicant: Bipha Corporation, Chitose-shi, Hokkaido 066-0051 (JP)
(72) Inventor: FUTAKI, Shiro, Nagaokakyo-shi, Kyoto 617-0823 (JP); SUGIURA, Yukio, Nishikyo-ku, Kyoto-shi, Kyoto 610-1146 (JP); KAMEYAMA, Shoju, Bipha Corporation, Chitose-shi, Hokkaido 066-0051 (JP); KIKUCHI, Takeo, Bipha Corporation, Chitose-shi, Hokkaido 066-0051 (JP)
(74) Representative: Keller, Günter, Dr.
(86) International application number: PCT/JP2003/003377
(87) International publication number: WO 2003/080115

(57) **Abstract**

The present invention provides an immunoglobulin preparation comprising an immunoglobulin-hydrophilic peptide complex in which an immunoglobulin and a hydrophilic peptide are linked optionally via a divalent group, and a pharmacologically acceptable carrier. The immunoglobulin preparation has cell permeability, or in other words, it can penetrate through a cell membrane and attain inside.

SEQUENCE LISTING

<110> BIPHA CORPORATION

<120> IMMUNOGLOBULIN-HYDROPHILIC PEPTIDE COMPLEX

<130> B06F1039

<160> 13

<210> 1

<211> 17

<212> PRT

<213> HIV-1 Rev-(34-50)

<400> 1

Thr Arg Gln Ala Arg Arg Asn Arg Arg Arg Arg Trp Arg Glu Arg Gln
1               5                   10                  15

Arg

<210> 2

<211> 15

<212> PRT

<213> FHV Coat-(35-49)

<400> 2

Arg Arg Arg Arg Asn Arg Thr Arg Arg Asn Arg Arg Arg Val Arg
1               5                   10                  15

<210> 3

<211> 19

<212> PRT

<213> BMV Gag-(7-25)

<400> 3

Lys Met Thr Arg Ala Gln Arg Arg Ala Ala Ala Arg Arg Asn Arg Trp

1               5                   10                  15

Thr Ala Arg

<210> 4

<211> 13

<212> PRT

<213> HTLV-II Rex-(4-16)

<400> 4

Thr Arg Arg Gln Arg Thr Arg Arg Ala Arg Arg Asn Arg

1               5                   10

<210> 5

<211> 19

<212> PRT

<213> CCMV Gag-(7-25)

<400> 5

Lys Leu Thr Arg Ala Gln Arg Arg Ala Ala Ala Arg Lys Asn Lys Arg

1               5                   10                  15

Asn Thr Arg

<210> 6

<211> 17

<212> PRT

<213> P22 N-(14-30)

<400> 6

Asn Ala Lys Thr Arg Arg His Glu Arg Arg Arg Lys Leu Ala Ile Glu

1               5                   10                  15

Arg

<210> 7

<211> 22

<212> PRT

<213> λN-(1-22)

<400> 7

Met Asp Ala Gln Thr Arg Arg Arg Glu Arg Arg Ala Glu Lys Gln Ala

1               5                   10                  15

Gln Trp Lys Ala Ala Asn

            20

<210> 8

<211> 18

<212> PRT

<213> φ21N-(12-29)

<400> 8

Thr Ala Lys Thr Arg Tyr Lys Ala Arg Arg Ala Glu Leu Ile Ala Glu
1               5                   10                  15
Arg Arg

<210> 9
<211> 16
<212> PRT
<213> Yeast PRP6-(129-144)
<400> 9
Thr Arg Arg Asn Lys Arg Asn Arg Ile Gln Glu Gln Leu Asn Arg Lys
1               5                   10                  15

<210> 10
<211> 8
<212> PRT
<213> (Arg)8
<400> 10
Arg Arg Arg Arg Arg Arg Arg Arg
1               5

<210> 11
<211> 28
<212> PRT
<213> Human cJun-(252-279)
<400> 11

Arg Ile Lys Ala Glu Arg Lys Arg Met Arg Asn Arg Ile Ala Ala Ser
1               5                   10                  15
Lys Ser Arg Lys Arg Lys Leu Glu Arg Ile Ala Arg
            20                  25

<210> 12
<211> 29
<212> PRT
<213> Human cFos-(139-164)analog
<400> 12
Arg Arg Arg Ile Arg Arg Ile Arg Arg Glu Arg Asn Lys Met Ala Ala
1               5                   10                  15
Ala Lys Ser Arg Asn Arg Arg Arg Glu Leu Thr Asp Thr
            20                  25

<210> 13
<211> 22
<212> PRT
<213> Yeast GCN4-(231-252)
<400> 13
Lys Arg Ala Arg Asn Thr Glu Ala Ala Arg Arg Ser Arg Ala Arg Lys
1               5                   10                  15
Leu Gln Arg Met Lys Gln
            20

## Description

### TECHNICAL FIELD

The present invention relates to an immunoglobulin-hydrophilic peptide complex, and a medicament comprising the same.

### BACKGROUND ART

Immunoglobulin comprises much amount of antibody against viruses, bacteria, etc., and is widely used as an immunoglobulin preparation for the prophylaxis or treatment of infectious diseases.

However, use of an immunoglobulin preparation has a problem that the immunoglobulin has not enough bioavailability and cannot exhibit its effect sufficiently, since an immunoglobulin cannot penetrate through cell membranes of viruses and bacteria, and of cells infected therewith.

### DISCLOSURE OF THE INVENTION

An object of the present invention is to provide an immunoglobulin preparation having cell permeability, i.e., capable of penetrating through a cell membrane and introducing an immunoglobulin into the cell.

The present inventors have conducted intensive studies in order to achieve the above-mentioned object, and have consequently found that an immunoglobulin can be introduced into a cell through a cell membrane by using an immunoglobulin-hydrophilic peptide complex in which an immunoglobulin and a hydrophilic peptide are linked optionally via a divalent group. Accordingly, a medicament comprising said immunoglobulin-hydrophilic peptide complex has higher bioavailability than that of conventional immunoglobulin preparations, and has a superior effect against infectious diseases, etc. The present inventors have carried out further investigations and thus completed the present invention.

Namely, the present invention relates to
(1) a medicament comprising an immunoglobulin-hydrophilic peptide complex in which an immunoglobulin and a hydrophilic peptide are linked optionally via a divalent group, and a pharmacologically acceptable carrier;
(2) the medicament according to the above (1), wherein the immunoglobulin is a polyclonal antibody and/or a monoclonal antibody;
(3) the medicament according to the above (2), wherein the immunoglobulin is a whole antibody or a variant thereof, or a part thereof;
(4) the medicament according to the above (1), wherein the immunoglobulin is IgG, IgA, IgD, IgE or IgM;
(5) the medicament according to the above (2), wherein the monoclonal antibody is a monoclonal antibody selected from the group consisting of the following (a) to (e) or a part thereof:
   (a) a natural antibody which can be obtained by immunization of a mammal, and a part thereof,
   (b) a chimeric antibody and a human antibody (CDR-grafted antibody), which can be produced by a genetic recombination technique, and a part thereof,
   (c) a human monoclonal antibody which can be produced by using a human antibody-producing transgenic animal, and a part thereof,
   (d) a monoclonal antibody which can be obtained from a monoclonal antibody-producing cell, and a part thereof,
   (e) a genetically engineered monoclonal antibody which can be obtained from a recombinant monoclonal antibody-producing cell, and a part thereof;
(6) the medicament according to the above (1) to (5), wherein the hydrophilic peptide is
   (a) a polypeptide represented by any one of SEQ ID Nos. 1 to 13,
   (b) a polypeptide having cell permeability comprising the above-mentioned polypeptide in which one or more of amino acids is/are substituted, deleted or added, or
   (c) a combination of the polypeptides selected from the group consisting of the above (a) and (b);
(7) the medicament according to the above (6), wherein the hydrophilic peptide has an -SH group in the side chain;
(8) the medicament according to the above (1) to (7), wherein the divalent group is -gly- or a group represented by the following formula: 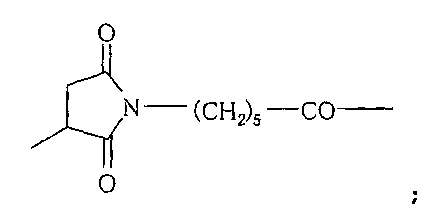
(9) a method for introducing an immunoglobulin into a cell, comprising bringing the immunoglobulin-hydrophilic peptide complex according to the above (1) into contact with a cell membrane to introduce the immunoglobulin into the cell;
(10) a method for enhancing bioavailability of an immunoglobulin, comprising administering the immunoglobulin-hydrophilic peptide complex according to the above (1) to a human or an animal other than human;
(11) an immunoglobulin-hydrophilic peptide complex; and
(12) a production method of an immunoglobulin-hydrophilic peptide complex in which an immunoglobulin and a hydrophilic peptide are linked optionally via a divalent group.

### BRIEF DESCRIPTION OF THE DRAWINGS

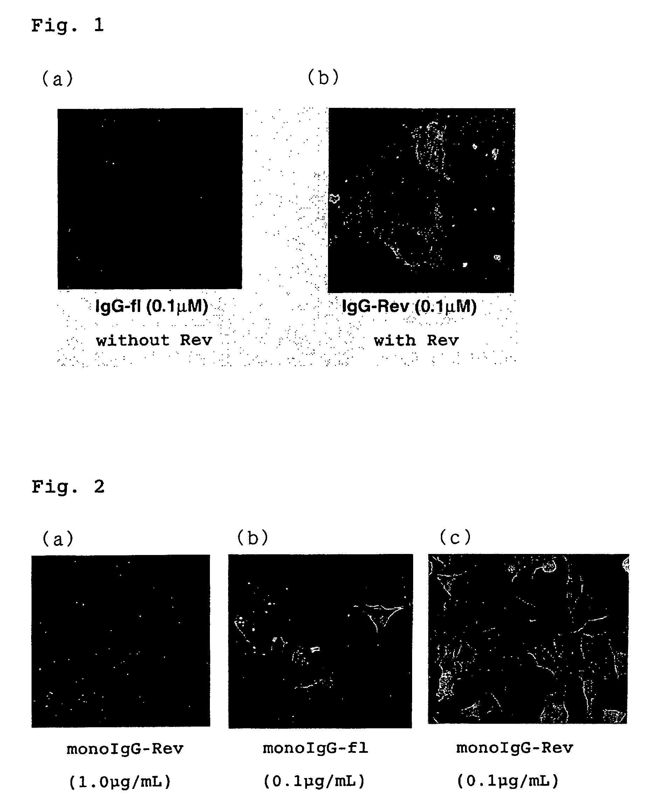

Fig. 1 (a) shows an MEM medium to which IgG-fl has been added to adjust the final concentration to 0.1 µM. Fig. 1 (b) shows an MEM medium to which IgG-Rev has been added to adjust the final concentration to 0.1 µM. In the case of the IgG-fl to which HIV-rev was not attached, uptake into cells was not observed. On the other hand, uptake into cells was observed in the case of IgG-Rev.
Fig. 2 (a) shows an MEM medium to which mono IgG-fl has been added to adjust the final concentration to 0.1 µM. Fig. 2 (b) shows an MEM medium to which mono IgG-Rev has been added to adjust the final concentration to 0.1 µM. Fig. 2 (c) shows an MEM medium to which mono IgG-Rev has been added to adjust the final concentration to 1 µM. In the case of the mono IgG-fl to which HIV-rev was not attached, uptake into cells was not observed. On the other hand, uptake into cells was observed in the case of the mono IgG-Rev.

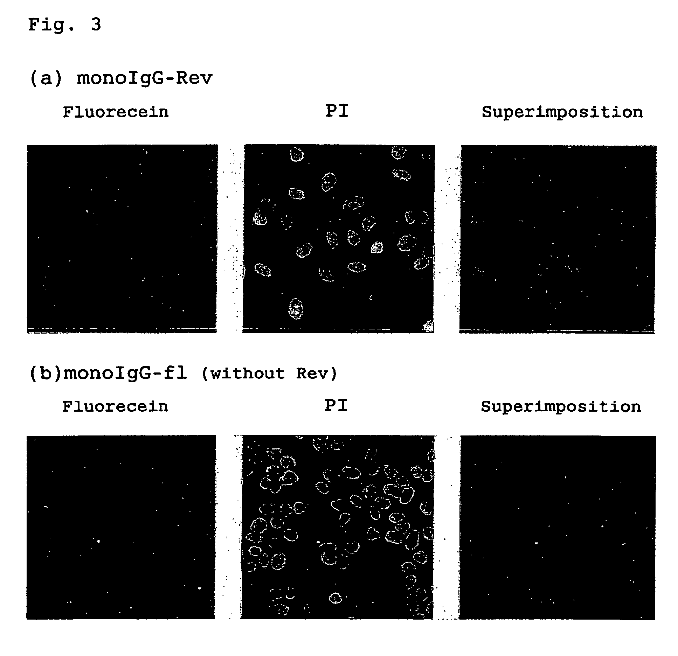

Fig. 3 shows the result of a confocal microscope observation. In the case of the mono IgG-fl, uptake into cells was not observed (b). On the other hand, uptake into cells was observed in the case of the mono IgG-Rev (a).

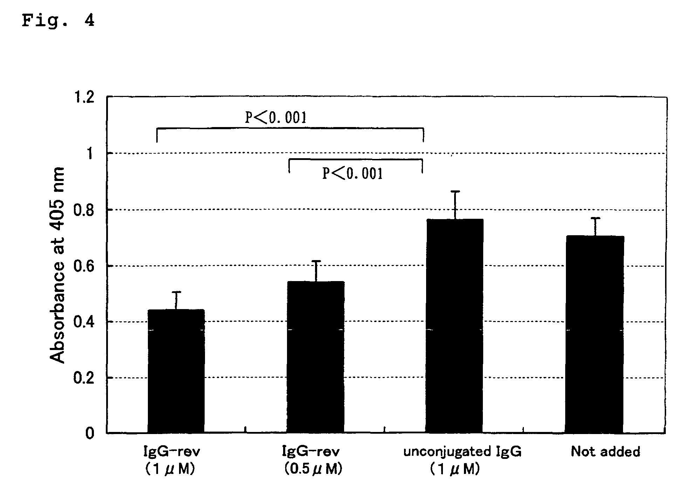

Fig. 4 shows the effects of IgG-rev and an unconjugated IgG on the cell proliferation of Hela cells. In the drawing, P<0.001 indicates that each of the IgG-rev (1 µM) and IgG-rev (0.5 µM) has a significant difference as compared with the unconjugated IgG in the t-test.

### BEST MODE FOR CARRYING OUT THE INVENTION

The present invention provides an immunoglobulin-hydrophilic peptide complex in which an immunoglobulin and a hydrophilic peptide are linked optionally via a divalent group.

The "hydrophilic peptide" is preferably a peptide comprising water soluble amino acid residues of about 10% ormore, preferably about 50% or more, and more preferably about 70% or more by the ration relative to the number of amino acid residues. The water soluble amino acid residues may exist sequentially or intermittently in the hydrophilic peptide, and preferably exist sequentially.

As used herein, the "peptide" means a single chain of amino acids linked by peptide bonds. The peptide consists of at least 2 amino acid residues, and generally consists of about not more than 50 amino acid residues. Specifically, the "hydrophilic peptide" used in the present invention preferably consists of about 6 to 25, preferably about 6 to 20, and more preferably about 7 to 15 amino acid residues.

The "water soluble amino acid" is not specifically limited as long as it has a polar group in the side chain. The "amino acid" in the "water soluble amino acid" may be of natural type or non-natural type. As used herein, the natural amino acid means amino acids constituting a natural (animal, plant or microorganism) protein or a metabolite thereof. The non-natural amino acid means amino acids other than natural amino acids. The amino acid may be in the L-form or D-form, or a mixture thereof. Furthermore, the amino acid may be an α-amino acid, a β-amino acid or a γ-amino acid, or a mixture of two or more of them.

Specifically, the "water soluble amino acid" includes amino acids having a polar group such as carboxyl group, amino group, guanidino group (-HN-C(=NH)(MH₂)), amidino group (-C(=NH)(NH₂)) or hydroxyl group, etc. in the side chain, and said amino acids are roughly classified into an acidic amino acid, a neutral amino acid or a basic amino acid depending on the kind of the side chain. Either of the amino acids may be used for the "water soluble amino acid" used in the present invention. Among these, a basic amino acid having a guanidino group (-HN-C(=NH)(NH₂)) or an amidino group (-C(=NH)(NH₂)) in the side chain is preferably used as the "water soluble amino acid". More specifically, said "water soluble amino acid" includes lysine (Lys), glutamine (Gln), aspartic acid (Asp), glutamic acid (Glu), threonine (Thr), asparagines (Asn), arginine (Arg), serine (Ser), histidine (His), ornithine, homoarginine, 2,3-diaminopropionic acid, 2,4-diaminobutylic acid, 2-amino-4-guanidinobutylic acid, 2-amino-3-guanidinopropionic acid, glycine (Gly), etc.

The "hydrophilic peptide" used in the present invention is preferably (a) a polypeptide represented by any one of SEQ ID NOs: 1 to 13, or (b) a polypeptide having cell permeability in which one or more of amino acids of said polypeptide is/are substituted, deleted or added, or (c) a combination of plural polypeptide selected from the group consisting of the above (a) and (b). In the case where the (c) is used, plural polypeptide may be selected from the polypeptides of (a) to be combined; or plural polypeptide may be selected from the polypeptides of (b) to be combined; or one or more of polypeptide(s) selected from (a) and one or more of polypeptide(s) selected from (b) may be combined.

In the case a polypeptide having cell permeability in which one or more of amino acids of a polypeptide represented by any one of SEQ ID NOs: 1 to 13 is/are substituted, deleted or added (b), the degree, location, etc. of the "deletion, substitution or addition of amino acids" are not specifically limited as long as amodified polypeptide has cell permeability. As used herein, "cell permeability" means that the immunoglobulin attached to the polypeptide permeates cell membranes and is introduced into cells. The number of the amino acid to be substituted, added or deleted is 1 to 2 or more, preferably about 1 to 10, and more preferably about 1 to 5. The amino acid to be substituted or added may be a non-natural amino acid that is not encoded by a gene.

The specific method for the substitution, addition or deletion may be a known method. For example, in the case where the method is carried out via a DNA encoding a polypeptide represented by any one of SEQ ID NOs: 1 to 13, there may be exemplified gene recombination techniques such as Site-specific Mutagenesis (Methods in Enzymology, 154, 350, 367-382 (1987); ditto 100, 468 (1983); NucleicAcidsRes., 12, 9441 (1984); Sequel Biochemistry Experiment Course 1 "Gene Investigation II", The Japan Biochemical Society ed, p. 105 (1986)), a chemical synthetic means such as a phosphate triester method, a phosphate amidite method, etc. (J. Am. Chem. Soc., 89, 4801 (1967); ditto 91, 3350 (1969); Science, 150, 178 (1968); Tetrahedron Lett.,22, 1859 (1981); ditto 24, 245 (1983)), and a combination method thereof. More specifically, the synthesis of DNA can be carried out by a chemical synthesis according to a phosphoramidite method or a triester method, or can be carried out using a commercially available automatic oligonucleotide synthesis apparatus. The double strand fragment can be obtained from chemically synthesized single strand product by synthesizing a complementary strand and annealing said strand under a suitable condition, or by using DNA polymerase together with a suitable primer sequence to add complementary strand thereto. Furthermore, the hydrophilic peptide of the present invention can be synthesized by solid phase synthesis using a peptide synthesis machine, and when such peptide synthesis machine is used, the substitution, addition or deletion can be readily carried out by changing the type of a protecting amino acid. Alternatively, non-natural amino acids such as D-amino acid, sarcosine (N-methylglycine), etc. can be introduced in the hydrophilic peptide of the present invention.

The "immunoglobulin" used in the present invention may be a polyclonal antibody (antiserum) or a monoclonal antibody. It is preferable that the immunoglobulin functions as an antibody when the immunoglobulin-hydrophilic peptide complex of the present invention is introduced into cells. The monoclonal antibody also comprises monoclonal antibodies having any of isotypes such as IgG, IgM, IgA, IgD, IgE, etc., preferably IgG, IgA or IgM. The IgG is preferably human IgG1, human IgG2, human IgG3 or human IgG4, and the IgA is preferably human IgA1 or human IgA2.

Alternatively, the "immunoglobulin" may be a domain of a part of the above-mentioned immunoglobulin, preferably a domain of a part of a monoclonal antibody. Specifically, F(ab')₂, Fab' , Fab, Fv (variable fragment of antibody), sFv, dsFv (disulphide stabilized Fv) or dAb (single domain antibody) , etc. (Exp. Opin. Ther. Patents, Vol.6, No.5, p.441-456, 1996) may be exemplified.

As used herein, the "F(ab')₂" and "Fab'" mean antibody fragments, which are produced by treating an immunoglobulin with a proteolytic enzyme, such as pepsin, papain, etc., wherein the immunoglobulin is digested at the upstream or downstream of the disulfide bond present between two H chains in the hinge region. For example, when IgG is treated with papain, it can be cleaved at the upstream of the disulfide bond present between two H chains in the hinge region to produce two homologous antibody fragments in which an L chain consisting of V_{L} (L chain variable domain) and C_{L} (L chain constant domain) and an H chain fragment consisting of V_{H} (H chain variable domain) and CHγ₁ (γ1 domain in H chain constant domain) are linked by a disulfide bond at the C-terminal domains. These two homologous antibody fragments are each referred to as Fab'.

Furthermore, when IgG is treated with pepsin, it can be cleaved at the low stream of the disulfide bond that exists between two H chains in the hinge region to give an antibody fragment slightly larger than the above-mentioned antibody fragment, in which two Fab's are linked with a hinge region. This antibody fragment is referred to as F(ab')₂.

The "immunoglobulin" used in the present invention may be a variant or a part of the immunoglobulin. The "variant of the immunoglobulin" includes an antibody comprising heavy chain and/or light chain, wherein 1 to several amino acid(s) is/are deleted, substituted or added in each of the amino acid sequences of the heavy chain and/or light chain that constitute the immunoglobulin. In general, such variants of the immunoglobulin have substantially the same biological properties as those of the pre-modified, natural type immunoglobulin before variation. As used herein, "several amino acids" means plural amino acids, specifically 1 to 10 amino acid(s), preferably 1 to 5 amino acid(s). The partial modification (deletion, substitution, addition) of the amino acid as mentioned above can be introduced into the amino acid sequence of the immunoglobulin by partially modifying the base sequence that encodes said amino acid sequence. The partial modification of the base sequence can be introduced by a known site specific mutagenesis according to a conventional method (Proc. Natl. Acsd. Sci. USA, Vol.81, p.5662-5666, 1984). The variant of the immunoglobulin used in the present invention is preferably a variant of a monoclonal antibody.

The "immunoglobulin" used in the present invention can be produced according to existing common production methods. Namely, for example, it can be produced by immunization of a mammal, preferably mouse, rat, hamster, guinea pig, rabbit, chicken, cat, dog, pig, goat, horse or cattle, more preferably mouse, rat, hamster, guinea pig or rabbit, with immunogen (antigen), optionally together with Freund's Adjuvant. A polyclonal antibody (antiserum) can be obtained from a serum obtained from said immune-sensitized animal.

The "monoclonal antibody" of the present invention includes natural antibodies obtained by immunization of a mammal such as mouse, rat, hamster, guinea pig, rabbit, etc., using immunogens; chimeric antibodies and a human antibodies (CDR-grafted antibodies), which can be produced by a genetic recombination technique; and a human monoclonal antibody produced, for example, by using a human antibody-producing transgenic animal, etc. Furthermore, the monoclonal antibody of the present invention includes a monoclonal antibody obtained from a monoclonal antibody-producing cell, or a genetically engineered monoclonal antibody obtained from a genetically engineered monoclonal antibody-producing cell, etc.

Such monoclonal antibody can be readily produced using a known method. A monoclonal antibody can be produced by, for example, preparing a hybridoma from a myeloma cell having no self-antibody-producing ability and an antibody-producing cell obtained from spleen, lymph node, bone marrow, blood (preferably peripheral blood) oramygdale, etc. , preferably, B cell of spleen of an immune-sensitized animal; cloning the hybridoma; and selecting the monoclonal antibody-producing clone that shows specific affinity against the immunogen used for the immunization of a mammal by an immunological measurement method such as ELISA, etc.

More specifically, the above-mentioned monoclonal antibody can be produced as described below. Namely, an immunogen, optionally together with Freund' s Adjuvant, is injected subcutaneously, intramuscularly, intravenously, in a foot pad or intraperitoneously one to several times or implanted to a mammal, preferably, mouse, rat, hamster, guinea pig, fowl or rabbit, more preferably, mouse, rat or hamster, to immunize the animal. In general, the immunization is carried out 1 to 4 time (s) at the intervals of about 1 to 14 day(s) from the initial immunization, and an antibody-producing cell can be obtained from said immune-sensitized mammal about 1 to 5 day(s) after the final immunization. The mammal includes a transgenic animal designed to produce an antibody derived from other animal, such as a human antibody-producing transgenic mouse.

The hybridoma that secretes a monoclonal antibody can be produced by a method according to Köhler and Milstein, et al. (Nature, Vol. 256, pp. 495 to 497, 1975) anda similarmodification method thereto. Namely, it is produced by cell fusion between an antibody-producing cell contained in spleen, lymph node, bone marrow, amygdala, etc., preferably spleen, of a mammal that is immune-sensitized as mentioned above and a myeloma cell having by itself no antibody-producing ability that is derived from preferably a mammal such as mouse, rat, guinea pig, hamster, rabbit, human, etc., more preferably from mouse, rat or human.

As the myeloma cell used for the cell fusion, for example, mouse myeloma P3/X63-AG8.653 (653; ATCC No. CRL1580), P3/NSI/1-Ag4-1 (NS-1), P3/X63-Ag8.U1 (P3U1), SP2/0-Ag14 (Sp2/0, Sp2), PAI, F0 or BW5147, rat myeloma 210RCY3-Ag.2.3., human myeloma U-266AR1, GM1500-6TG-A1-2, UC729-6, CEM-AGR, D1R11 or CEM-T15 can be used.

The monoclonal antibody-producing hybridoma clone can be screened, for example, by culturing hybridoma in a microtiter plate and measuring the reactivity of the culture supernatant of a well in which proliferation is observed, against immunizing antigen by an enzyme immunization measurement method such as RIA, ELISA, etc. A monoclonal antibody can be obtained in vitro from a hybridoma by isolation from a culture supernatant, or can be obtained in vivo from an abdominal dropsy of a mammal, for example, mouse, rat, guinea pig, hamster or rabbit, etc., preferably, mouse or rat, more preferably, mouse. In the case where the cultivation is carried out in vitro, the hybridoma may be proliferated, maintained and preserved using a known nutrition medium used for the production of a monoclonal antibody in a culture supernatant or any nutrition medium derived and prepared from a known base medium depending on various conditions such as the property of cells to be cultured and the cultivation method.

The base medium includes, for example, low calcium media such as Ham's F12 medium, MCDB153 medium, low calcium MEM medium, etc., or high calcium media such as MCDB104 medium, MEM medium, D-MEM medium, RPMI1640 medium, ASF104 medium, RD medium, etc. The base medium can include, for example, serum, hormone, cytokine and/or various inorganic or organic substance, etc. depending on the purpose.

The isolation and purification of a monoclonal antibody can be carried out by subjecting the above-mentioned culture supernatant or abdominal dropsy to saturated ammonium sulfate, euglobulin precipitation method, caproic acid method, caprylic acid method, ion exchange chromatography (DEAE, DE52, etc.), affinity column chromatography such as anti-immunoglobulin column, protein A column, etc. Also, there is a method where a transgenic animal (e.g., goat, sheep, pig) is produced in such a manner that a monoclonal antibody-encoding gene is cloned from the hybridoma, and the antibody-encoding gene is incorporated in the endogenous gene using a technology for producing a transgenic animal, and then a monoclonal antibody derived from said antibody gene can be obtained in large amounts from milk, etc. of the transgenic animal (Nikkei Science, April 1997, pp. 78 to 84).

The immunoglobulin of the present invention may be a recombinant chimeric monoclonal antibody. The "recombinant chimeric monoclonal antibody" is a monoclonal antibody produced by genetic engineering, and specifically includes, for example, a chimeric monoclonal antibody such as a murine/human chimeric monoclonal antibody, etc. in which the variable domain is a murine immunoglobulin variable domain and the constant domain is a human immunoglobulin constant domain. The human immunoglobulin constant domain each has an inherent amino acid sequence depending on the isotype such as IgG, IgM, IgA, IgD, IgE, etc., while the constant domain of the recombinant chimeric monoclonal antibody of the present invention may be any constant domain of human immunoglobulin belonging to any isotype, or preferably, a constant domain of human IgG.

The above-mentioned chimeric monoclonal antibody can be prepared according to a known method. For example, a murine/human chimeric monoclonal antibody can be prepared with reference to Experimental Medicine (provisional extra number), Vol. 1.6, No. 10, 1988, and Japanese Patent Application Publication No. H3-73280, etc.

The immunoglobulin of the present invention may be a human antibody (CDR-grafted antibody). The "human monoclonal antibody" of the present invention is a monoclonal antibody prepared by genetic engineering. Specifically, there may be exemplified a human monoclonal antibody in which a part or all of the complementarity-determining region of the hypervariable region is a complementarity-determining region of a hypervariable region derived from a monoclonal antibody of a non-human mammal (mouse, rat, hamster, etc.); the frame region of the variable region is a frame region of a variable region derived from human immunoglobulin; and the constant region is a constant region derived from human immunoglobulin.

As used herein, the complementarity-determining region of the hypervariable region means three regions (complementarity-determining residue; CDR1, CDR2 and CDR3), which exist in the hypervariable region of the variable region of the antibody and bind complementarily and directly to an antigen, and the frame region of the variable region means four regions (Framework; FR1, FR2, FR3 and FR4), which intervene in the vicinity of the three complementarity-determining regions and are relatively reserved. In other words, a monoclonal antibody in which all domains other than a part or the entire complementarity-determining region of the hypervariable region of the monoclonal antibody derived from a non-human mammal have been replaced with the corresponding regions of the human immunoglobulin can be exemplified. Although the human immunoglobulin constant regions each has an inherent amino acid sequence depending on the isotype such as IgG (IgG1, IgG2, IgG3, IgG4), IgM, IgA, IgD, IgE, etc. , the constant region of the human monoclonal antibody of the present invention may be a constant region of a human immunoglobulin belonging to any isotype, preferably, a constant region of human IgG. In addition, with regard to the frame region of the human immunoglobulin variable domain, the isotype thereof is not limited.

The human monoclonal antibody of the present invention can be produced according to a known method. For example, a recombinant human monoclonal antibody derived from a murine monoclonal antibody can be produced by genetic engineering with reference to Japanese Patent National Publication No. 4-506458 and Japanese Patent Land-Open No. 62-296890, etc.

The immunoglobulin of the present invention may be a human monoclonal antibody that can be produced using a human antibody-producing transgenic animal. The human monoclonal antibody can be produced, for example, by immunization of "a transgenic non-human mammal capable of producing a human antibody" as typified by "a transgenicmouse capable of producing a human antibody" using an immunogen. The immunization of the transgenic non-human mammal, preparation and screening of the antibody-producing fusion cell (hybridoma), and mass production of the human monoclonal antibody can be carried out using the above-mentioned common methods. (Nature Genetics, Vol. 7, p. 13-21, 1994; Nature Genetics, Vol. 15, p. 146-156, 1997; Japanese Patent National Publication No. 4-504365; Japanese Patent National Publication No. 7-509137; Nikkei Science, issued in June, pp. 40-50, 1995; WO 94/25585; Nature, Vol.368, p.856-859, 1994; and Japanese Patent National Publication No. 6-500233, etc.)

Specifically, the human antibody-producing transgenic mouse can be produced, for example, using the procedure comprising the following steps. Other human antibody-producing transgenic non-human mammals can be produced in a similar manner.
(1) A step of substituting at least a part of a murine endogenous immunoglobulin heavy chain gene locus with a drug resistant marker gene (neomycin resistant gene, etc.) by homologous recombination so as to produce a knockout mouse in which the murine endogenous immunoglobulin heavy chain gene is functionally inactivated.
(2) A step of substituting at least a part of a murine endogenous immunoglobulin light chain gene locus with a drug resistant marker gene (neomycin resistant gene, etc) by homologous recombination so as to produce a knockout mouse in which the murine endogenous immunoglobulin light chain gene (specifically, κ chain gene) is functionally inactivated.
(3) A step of using a vector that can convey a macrogene such as a yeast artificial chromosome (YAC) vector, etc. to produce a transgenic mouse in which the desired region on a human immunoglobulin heavy chain gene locus is incorporated in a murine chromosome.
(4) A step of using a vector that can convey a macrogene such as YAC, etc., to produce a transgenic mouse in which the desired region on a human immunoglobulin light chain (specifically, κ chain) gene locus has been incorporated in a murine chromosome.
(5) A step for cross-fertilizing the knockout mouse and a transgenic mouse according to the above-mentioned (1) to (4) in discretionary order to produce a transgenic mouse in which both of the murine endogenous immunoglobulin heavy chain gene locus and the murine endogenous immunoglobulin light chain gene locus are functionally inactivated and both of the desired region on the human immunoglobulin heavy chain gene locus and the desired region of the human immunoglobulin light chain gene locus are incorporated in a murine chromosome.

The above-mentioned knockout mouse can be produced by substituting a suitable region on a murine endogenous immunoglobulin gene locus with an exotic marker gene (neomycin resistant gene, etc.) by homologous recombination in order to inactivate the gene locus for preventing rearrangement of the gene locus. The inactivation using the homologous recombination can be carried out by, for example, using a method referred to as Positive Negative Selection (PNS) (Nikkei Science, issued in May, p.52-62, 1994). The functional inactivation of the immunoglobulin heavy chain gene locus can be achieved by, for example, introducing a disorder in a part of J region or C region (e.g., Cµ domain). Alternatively, the functional inactivation of the immunoglobulin light chain (e.g., κ chain) can be achieved by, for example, introducing a disorder in a part of J region or C region, or in the region comprising a domain cutting across the J region and the C region.

The transgenic mouse can be produced according to common methods generally used for the production of a transgenic animal (e.g., see Latest Animal cell Experimental Manual, issued by L. I. C., Section 7, pp. 361 to 408, 1990). Specifically, for example, HPRT negative (i.e. lacking hypoxanthine guanine-phosphoribosyltransferase gene) ES cell (embryonic stem cell) derived from normal murine blastcyst is fused to a yeast comprising a gene encoding the human immunoglobulin heavy chain gene locus or light chain gene locus or a part thereof and YAC vector to which an HPRT gene is inserted by spheroplast fusion method. The ES cell in which the exotic gene is integrated on the murine endogenous gene is selected by HAT selection method. The selected ES cell is then microinjected to a fertilized ovum (blastcyst) obtained from another normal mouse (Proc. Natl. Acad. Sci. USA, Vol.77, No.12, pp.7380-7384, 1980; U.S.Patent No. 4,873,191). The blastcyst is implanted in the uterine of another normal mouse as a foster parent. As such, a chimeric transgenic mouse is born from the foster parent mouse. A heterotransgenic mouse can be obtained by cross-fertilization of the chimeric transgenic mouse and a normal mouse. A homogeneic transgenic mouse can be obtained by cross-fertilization of the same heterogeneic transgenic mice according to the Mendel's law.

The "monoclonal antibody-producing cell" or "recombinant monoclonal antibody-producing cell" of the present invention can include any cell that produces the above-mentioned monoclonal antibody of the present invention. Specifically, cells as described in the following (1) to (3) can be exemplified.
(1) A monoclonal antibody-producing B cell, which can produce a monoclonal antibody showing reactivity against the objective antigen of the immunoglobulin preparation of the present invention and can be obtained from a mammal other than human or a transgenic mouse (or other transgenic non-human mammal) capable of producing the above-mentioned human antibody.
(2) The above-mentioned fusion cell (hybridoma) obtained by cell fusion of the thus-obtained antibody-producing B cell and a mammal-derived myeloma cell.
(3) A monoclonal antibody-producing transformed cell (genetically engineered cell) obtained by transforming the B cell and a cell other than hybridoma (e.g., CHO (Chinese hamster ovarian) cell, BHK (baby hamster kidney) cell, etc.) with the gene encoding monoclonal antibody (one or both of the gene encoding heavy chain or the gene encoding light chain) isolated from the monoclonal antibody-producing B cell or the monoclonal antibody-producing fusion cell (hybridoma).

As used herein, the monoclonal antibody-producing transformed cell (genetically engineered cell) mentioned in the above (3) means a genetically engineered cell that produces genetically engineered form of the monoclonal antibody produced by the B cell mentioned in the above (1) or the hybridoma of the above (2). These antibody-producing transformed cells can be produced by common genetic recombination techniques used for the production of the above-mentioned chimeric monoclonal antibody and the human monoclonal antibody.

The immunoglobulin-hydrophilic peptide complex of the present invention can be prepared by linking the above-mentioned hydrophilic peptide and immunoglobulin. In the case where the hydrophilic peptide has a cystein residue, the immunoglobulin can be linked to the cystein residue of the hydrophilic polypeptide via an -SS- bond. It is preferable to link the hydrophilic peptide and the immunoglobulin via an -SS- bond in this manner, since the -SS- bond is reduced in cells to release the immunoglobulin. Accordingly, the hydrophilic peptide preferably has a -SH group in the side chain.

Alternatively, the hydrophilic peptide and the immunoglobulin may be linked via a crosslinker. The crosslinker is not specifically limited as long as it is an at least divalent crosslinker that can link the hydrophilic peptide and the immunoglobulin of the present invention, and the example thereof includes N-(6-maleimidecaproyloxy)succinimide ester (EMCS), etc. When the EMCS is used as a crosslinker, the divalent group that links the hydrophilic peptide and the immunoglobulin of the present invention is a group represented by the following formula: 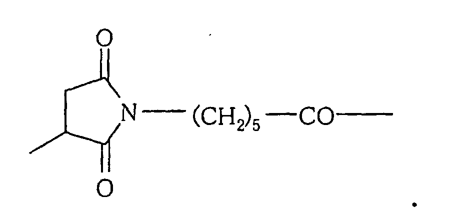 In addition, the divalent group that links the hydrophilic peptide and the immunoglobulin of the present invention includes -gly-, etc.

Preferably, a Cys residue is further linked to the C-terminal side of the hydrophilic peptide of the present invention, for example, in a manner such as Cys, Gly-Cys, etc. The SH group of the Cys residue is useful for addition reaction to the maleimide group of EMCS and for linking of the hydrophilic peptide and the immunoglobulin of the present invention via an -SS- bond when the immunoglobulin has a free SH group.

Additionally, an immunoglobulin-hydrophilic peptide complex in which the immunoglobulin has been linked directly to the C-terminal side of the hydrophilic peptide of the present invention can be obtained according to a common method, for example, by linking, preferably directly linking, a polynucleotide encoding the hydrophilic peptide of the present invention and a polynucleotide (gene) encoding the immunoglobulin to be introduced thereto, introducing the linked polynucleotides into a vector and expressing in a host cell such as Escherichia coli, yeast or an animal cell (e.g. CHO cell, etc.).

The medicament according to the present invention is characterized by comprising the above-mentioned immunoglobulin-hydrophilic peptide complex and a pharmacologically acceptable carrier. The medicament according to the present invention has an advantage that the bioavailability of the immunoglobulin is improved compared to that of the conventional immunoglobulin preparations, since a hydrophilic peptide having cell permeability is linked to the immunoglobulin and the thus immunoglobulin is introduced in cells penetrating through a cell membrane.

The "pharmaceutically acceptable carrier" is not specifically limited as long as it is a carrier known *per se* or conventionally used in the art of drug preparation, and the examples thereof include an excipient, a diluent, a filler, a disintegrator, a stabilizer, a preservative, a buffer, an emulsifier, a fragrance, a colorant, a sweetener, a thickener, a corrigent, a solubilizer or other additives, etc. Alternatively, for example, when the medicament of the present invention is in the form of an ointment, oil bases such as vaseline, petrolatum, silicone, vegetable oil, etc.; emulsion bases such as hydrophilic vaseline, refined lanoline, etc. ; base materials such as water soluble bases (e.g. macrogol); emulsifiers such as anionic or nonionic surfactant and preservatives such as paraoxybenzoic acid esters can also be included in the "pharmaceutically acceptable carrier".

The form of the medicament of the present invention is not specifically limited, and may be in the form of tablet (inclusive of coated tablet such as sugar coated tablet, enteric coated tablet, etc. or multilayer tablet), pill, powder, granule, injection, liquid preparation, capsule (inclusive of enteric coated capsule), troche, elixir, suspension, emulsion, syrup, etc. The medicament of the present invention may be in the form of external preparation, suppository for enteric administration, pessary, etc. The external preparation may include, for example, transdermal administration preparations such as ointment, patch, cataplasm, cream, plaster, tape, lotion, liquid preparation, suspension, emulsion, spray, etc.

The administration route of the medicament of the present invention is not specifically limited, and the medicament can be administered orally or parenterally depending on the above-mentioned form of the medicament of the present invention. For example, in the case where the medicament of the present invention is an injection, there may be exemplified medically suitable administration forms such as intravenous injection, subcutaneous injection, intradermal injection, intramuscle injection or intraperitoneal injection.

The application of the medicament of the present invention is not specifically limited. Immunoglobulin has anti-pathogenic microorganism effects such as microbicidal effect, microbial proliferation-suppressing effect, etc. As used herein, the microorganism is not specifically limited, and includes, for example, germ such as virus, bacteria, etc., fungi such as mold, etc., parasites, etc. Accordingly, the medicament of the present invention is preferably used as a prophylactic and/or therapeutic drug for various infectious diseases by effectively utilizing such effects of immunoglobulin. Specifically, the medicament of the present invention is more preferably used as a prophylactic and/or therapeutic drug for infectious diseases caused by germs or viruses in liver such as hepatitis virus, infectious diseases caused by germs or viruses in kidneys such as nephritis virus, etc.

The dose of the medicament of the present invention to be administered is not generally determined since the amount varies depending on the kind of disease to be treated, symptom and seriousness of the disease, age, sex or body weight of a patient, administration method, the kind of immunoglobulin, etc. For example, in the case of local administration, the dose is preferably about 1 ng to 10 mg per an administration. In the case of systemic administration, the dose is preferably about 1 µg to 10 g/kg per an administration.

### EXAMPLES

### Example 1

### (1) Preparation of IgG-Rev conjugate

N-(6-maleimidocaproyloxy)succinimide (80 µg) (Dojin Chemical Co., Ltd.; hereinafter abbreviated as EMCS,) which is a bifunctional crosslinker and 305 µg of fluorescein-5(6)-carboxamidocaproic acid N-hydroxy succinimide ester were added to 300 µg of a solution of human polyclonal IgG (purchased from Mitsubishi Pharma Corporation; concentration was adjusted to about 3 mg/ml), and the mixture was stirred gently at room temperature for 2 hours. The reaction mixture was concentrated to about 5 µL using an ultrafiltration membrane (Microcon YM-50; nominal molecular weight limit (NMWL): 50,000, centrifugation: 14,000 rpm, 20°C, 12 min) to remove unreacted EMCS and fluorescein-5(6)-carboxamidocaproic acid N-hydroxy succinimide ester.

After 300 µL of PBS (-) buffer was added to the above concentrate andmixed, 670 µg of HIV-revpeptidewas added thereto, and the mixture was stirred gently at room temperature for 1.5 hours. To the resulting mixture, 20 µL of Tris buffer (0.1 M, pH 8.0) comprising 6M Guanidine-HCL was added to suppress the aggregation of the reaction product. The thus obtained reaction solution was concentrated to about 5 µL using an ultrafiltration membrane (Microcon YM-50; NMWL: 50,000, centrifugation: 14,000 rpm, 20°C, 12 min) to remove unreacted HIV-rev peptide. The concentration was examined from the fluorescence intensity, and the introduction of HIV-rev peptide was confirmed by 15% SDS-PAGE.

The product labeled solely by fluorescence is referred to as IgG-fl, and the product to which HIV-rev has been further bonded is referred to as IgG-Rev.

### (2) Confirmation of uptake of Poly IgG-Rev into cell

Hela cells (10⁵ cells/mL) were dispensed in an amount of about 240 µL in each of the wells on a chamber slide (manufactured by Nunc, Inc., Lab-tech-II), and were cultured under the condition of 37°C and 5% CO₂ for 48 hours. The slide was washed twice with PBS (-) buffer, and the solution was exchanged with about 240 µL of fresh MEM medium, and then an MEM medium to which IgG-fl or IgG-Rev was added to adjust the final concentration to 0.1 µM was added thereto. After cultivation under the condition of 37°C and 5% CO₂ for 1 hour, followed by washing three times with PBS(-) buffer, the cells were fixed using a mixed solution of acetone:methanol (1:1) for 40 seconds. The cells were washed twice with PBS (-) buffer, and PPD glycerol and a cover glass were placed on each well, and then uptake into cells was observed using a fluorescent microscope.

As a result, uptake into cells was not observed in the case of the IgG-fl to which HIV-rev has not been bonded, whereas uptake into cells was observed in the case of IgG-Rev (Fig. 1).

### Example 2

### (1) Preparation of monoclonal antibody IgG-Rev conjugate

A solution of a murine monoclonal antibody against cdk1 (also referred to as cdc2) (manufactured by Lab Vision Corporation), which is important for the progression of cell cycle, was adjusted to about 3 mg/mL using PBS(-) buffer. To this solution (300 µL), 80 µg of N-(6-maleimidocaproyloxy) succinimide (Dojin Chemical Co., Ltd.; hereinafter abbreviated to as EMCS) which is a bifunctional crosslinker, and 305 µg of fluorescein-5(6)-carboxamidocaproic acid N-hydroxy succinimide ester were added, and the mixture was stirred gently at room temperature for 2 hours. The reaction solution was concentrated to about 5 µL using an ultrafiltration membrane (Microcon YM-50; NMWL: 50,000, centrifugation: 14,000 rpm, 20°C, 12 min) to remove the unreacted EMCS and fluorescein-5(6)-carboxamidocaproic acid N-hydroxy succinimide ester.

After 300 µL of PBS (-) buffer was added to the above concentrate and mixed, 670 µg of HIV-rev peptide was added thereto, and the mixture was stirred gently at room temperature for 1.5 hours. To the resulting mixture, 20 µL of Tris buffer (0.1 M, pH 8.0) comprising 6 M Guanidine-HCL was added to suppress the aggregation of the reaction product. The thus obtained reaction solution was concentrated to about 5 µL using an ultrafiltration membrane (Microcon YM-50; NMWL 50,000, centrifugation 14,000 rpm, 20°C, 12 min) to remove unreacted HIV-rev peptide. The concentration was examined from fluorescence intensity and the introduction of HIV-rev peptide was confirmed by 15% SDS-PAGE.

The product labeled solely by fluorescence is referred to as mono IgG-fl, and the product to which HIV-rev has been further bonded is referred to as mono IgG-Rev.

### (2) Confirmation of uptake of mono IgG-Rev into cell

Hela cells (10⁵ cells/mL) were dispensed in an amount of about 240 µL in each of the wells on a chamber slide (manufactured by Nunc, Inc., Lab-tech-II), and were cultured under the condition of 37°C and 5% CO₂ for 48 hours. The slide was washed twice with PBS (-) buffer, and further washed with about 240 µL of fresh MEM medium, and then an MEM medium to which mono IgG-fl or mono IgG-Rev was added to adjust the final concentrations to 0.1 µM and 1 µM was added thereto. After cultivation under the condition of 37°C and 5% CO₂ for 1 hour, followed by washing three times with PBS (-) buffer, the cells were fixed using a mixed solution of acetone: methanol (1:1) for 40 seconds. The cells were washed twice with PBS (-) buffer, and PPD glycerol and a cover glass were placed on each well, and then the uptake into cells was observed using a fluorescent microscope.

As a result, uptake into cells was not observed in the case of the mono IgG-fl to which HIV-rev has not been bonded, whereas uptake into cells was observed in the case of mono IgG-Rev (Fig. 2). On the other hand, in the confocal microscope observation, uptake into cells was not observed in the case of the mono IgG-fl, whereas uptake into cells was observed in the case of mono IgG-Rev (Fig. 3).

### Example 3

Attachment of Rev to IgG enables not only introducing IgG molecule into cells but also exhibiting the functions of the IgG molecule as an antibody in cells.

For example, the pharmacological effect of introduction of an antibody into cells can be confirmed by investigation of the effect on the cell proliferation using an antibody against cdc25c (anti-cdc25c antibody) which is an extremely important factor for the cell cycle. An experimental example where Hela cells are used is shown below. Hela cells (3×10⁴ cells/mL) in logarithmic growth phase were seeded on a 96-well plate in an amount of 100 µL per a well. The cells were pre-cultured under the condition of 37°C and 5% CO₂ overnight, and then the medium was replaced with a fresh medium (100 µL). Each 25 µL of unconjugated anti-cdc25c antibody or Rev-conjugated anti-cdc25c antibody was added thereto and cultured under the condition of 37°C and 5% CO₂ for 24 hours, and further cultured for 24 hours in a medium free from agents. Next, cell proliferation ability was measured with WST-8 kit for measuring cell proliferation (manufactured by Kishida Chemical Co., Ltd.). Specifically, 10 µL of WST-8 solution was added to each well, cultured under the condition of 37°C and 5% CO₂ for 4 hours, and the absorbance at 450 nm was measured. The change of absorbance is proportional to the number of living cells, indicating that the higher the absorbance value is, the higher the cell proliferation is. The result is shown in Fig. 4. Since the anti-cdc25c antibody to which Rev was not attached (unconjugated IgG) could not be transferred into cells, it showed similar cell proliferation to that of the group free of antibody and showed no effect, whereas the anti-cdc25c antibody in which Rev was attached (IgG-rev) suppressed cell proliferation in dose-dependent manner. Namely, it is shown that the anti-cdc25c antibody to which rev was attached was introduced into a cell and exhibited its function in the cell.

### INDUSTRIAL APPLICABILITY

When the immunoglobulin-hydrophilic peptide complex of the present invention is used, the immunoglobulin can be introduced into a cell of microorganism, etc. penetrating through a cell membrane. Accordingly, the medicament comprising the immunoglobulin-hydrophilic peptide complex of the present invention has high bioavailability and superior anti-microbial effect, and is specifically effective as a prophylactic or therapeutic drug for infectious diseases.

## Claims

1. A medicament comprising an immunoglobulin-hydrophilic peptide complex in which an immunoglobulin and a hydrophilic peptide are linked optionally via a divalent group, and a pharmacologically acceptable carrier.

2. The medicament according to Claim 1, wherein the immunoglobulin is a polyclonal antibody and/or a monoclonal antibody.

3. The medicament according to Claim 2, wherein the immunoglobulin is a whole antibody or a variant thereof, or a part thereof.

4. The medicament according to Claim 1, wherein the immunoglobulin is IgG, IgA, IgD, IgE or IgM.

5. The medicament according to Claim 2, wherein the monoclonal antibody is a monoclonal antibody selected from the group consisting of the following (a) to (e) or a part thereof:
(a) a natural antibody which can be obtained by immunization of a mammal, and a part thereof,
(b) a chimeric antibody and a human antibody (CDR-grafted antibody), which can be produced by a genetic recombination technique, and a part thereof,
(c) a human monoclonal antibody which can be produced by using a human antibody-producing transgenic animal, and a part thereof,
(d) a monoclonal antibody which can be obtained from a monoclonal antibody-producing cell, and a part thereof,
(e) a genetically engineered monoclonal antibody which can be obtained from a recombinant monoclonal antibody-producing cell, and a part thereof.

6. The medicament according to Claims 1 to 5, wherein the hydrophilic peptide is
(a) a polypeptide represented by any one of SEQ ID Nos. 1 to 13,
(b) a polypeptide having cell permeability comprising the above-mentioned polypeptide in which one or more of amino acids is/are substituted, deleted or added, or
(c) a combination of the polypeptides selected from the group consisting of the above (a) and (b).

7. The medicament according to Claim 6, wherein the hydrophilic peptide has an -SH group in the side chain.

8. The medicament according to Claims 1 to 7, wherein the divalent group is -gly- or a group represented by the following formula:

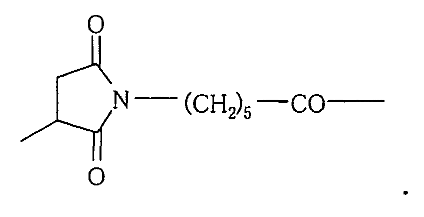

9. A method for introducing an immunoglobulin into a cell, comprising bringing the immunoglobulin-hydrophilic peptide complex according to Claim 1 into contact with a cell membrane to introduce the immunoglobulin into the cell.

10. A method for enhancing bioavailability of an immunoglobulin, comprising administering the immunoglobulin-hydrophilic peptide complex according to Claim 1 to a human or an animal other than human.

11. An immunoglobulin-hydrophilic peptide complex.

12. A production method of an immunoglobulin-hydrophilic peptide complex in which an immunoglobulin and a hydrophilic peptide are linked optionally via a divalent group.
